# EUROPÄISCHE PATENTSCHRIFT

(11) **EP 2 485 707 B1**
(45) Veröffentlichungstag und Bekanntmachung des Hinweises auf die Patenterteilung: **06.09.2017**
(21) Anmeldenummer: 10751784.9
(22) Anmeldetag: 14.07.2010
(51) Int. Cl.: A61K 8/34, A61Q 19/00

(54) **VERWENDUNG VON 4-N-BUTYLRESORCIN ZUR HERSTELLUNG EINER KOSMETISCHEN ODER DERMATOLOGISCHEN ZUBEREITUNG ZUR PROPHYLAXE ODER BEHANDLUNG VON AUGENRINGEN DIE DURCH EINE GESTÖRTE DURCHBLUTUNG DER HAUT IM AUGENBEREICH HERVORGERUFEN WERDEN**
USE OF 4-N-BUTYLRESORCINOL TO PRODUCE A COSMETIC OR DERMATOLOGICAL PREPARATION FOR PREVENTING OR TREATING UNDER-EYE CIRCLES THAT ARE CAUSED BY DISRUPTED BLOOD FLOW THROUGH THE SKIN IN THE EYE AREA
UTILISATION DE 4-N-BUTYLRÉSORCINE POUR PRODUIRE UNE PRÉPARATION COSMÉTIQUE OU DERMATOLOGIQUE POUR PRÉVENIR OU TRAITER LES CERNES PROVOQUÉS PAR UNE CIRCULATION SANGUINE CUTANÉE DÉFAILLANTE DANS LA ZONE DES YEUX.

(30) Priorität: 09.10.2009 DE 102009048970
(43) Veröffentlichungstag der Anmeldung: 15.08.2012
(73) Patentinhaber: Beiersdorf AG, 20253 Hamburg (DE)
(72) Erfinder: KOLBE, Ludger, 21255 Dohren (DE); NEUFANG, Gitta, 20149 Hamburg (DE); IMMEYER, Jeannine, 21272 Egestorf-Sahrendorf (DE); SCHERNER , Cathrin, 22844 Nordestedt (DE)
(86) Internationale Anmeldenummer: PCT/EP2010/004271
(87) Internationale Veröffentlichungsnummer: WO 2011/042076

(56) Entgegenhaltungen:
- EP-A1- 1 543 825
- WO-A1-03/080009
- WO-A1-2008/148968

## Beschreibung

Die vorliegende Erfindung betrifft die Verwendung von 4-n-Butylresorcin zur Herstellung einer kosmetischen oder dermatologischen Zubereitung zur Prophylaxe oder Behandlung von Augenringen die durch eine gestörte Durchblutung der Haut im Augenbereich hervorgerufen werden.

Übliche kosmetische Darreichungsformen sind Emulsionen. Darunter versteht man im allgemeinen ein heterogenes System aus zwei miteinander nicht oder nur begrenzt mischbaren Flüssigkeiten, die üblicherweise als Phasen bezeichnet werden. Die eine liegt dabei in Form von Tröpfchen vor (dispere oder innere Phase), während die andere Flüssigkeit eine kontinuierliche (kohärente oder innere Phase) bildet. Seltenere Darreichungsformen sind multiple Emulsionen, also solche, welche in den Tröpfchen der dispergierten (oder diskontinuierlichen) Phase ihrerseits Tröpfchen einer weiteren dispergierten Phase enthalten, z.B. W/O/W-Emulsionen und O/W/O-Emulsionen.

Neuere Erkenntnisse führten in letzter Zeit zu einem besseren Verständnis praxisrelevanter kosmetischer Emulsionen. Dabei geht man davon aus, daß die im Überschuß eingesetzten Emulgatorgemische lamellare flüssigkristalline Phasen bzw. kristalline Gelphasen ausbilden. In der Gelnetzwerktheorie werden Stabilität und physikochemische Eigenschaften solcher Emulsionen auf die Ausbildung von viskoelastischen Gelnetzwerden zurückgeführt.

Kosmetische Zubereitungen mit 4-n-Butylresorcin sind bekannt, beispielsweise aus der EP 1 490 017.

4-n-Butylresorcin wird auch Rucinol oder Lucinol genannt. Es hemmt die Melaninproduktion, indem es das für die Melaninsynthese (Melanogenese) erforderliche Enzym, die Tyrosinase, hemmt. Es wird zunächst die Melaninproduktion gehemmt, dann die Produktion des schwarzen Melanins, das für die intensive Färbung der Pigmentflecken verantwortlich ist, blockiert.

Augenringe entstehen als Reaktion auf allgemeinen Stress, wie z.B. wenig Schlaf oder schlicht durch Überanstrengung der Augen. Bei jüngeren Menschen verschwinden die Symptome nach ausreichender Nachtruhe wieder, über längere Zeiträume jedoch kann der Zustand chronisch und für die betroffenen Personen sehr störend werden.
Physiologisch verbirgt sich dahinter ein veränderter Blutfluss in den kleinen Gefäßen der Haut um die Augen. Im Augenbereich ist die Haut besonders dünn, so dass ein verringerter Rückfluss des Bluts sichtbar wird. Da venöses Blut eine bläulich-rote Farbe hat äußert sich das als dunkle Augenringe.

Augenringe können auch als Folgen einer Pigmentierungsstörung entstehen. Derlei Augenring sind jedoch nicht Gegenstand der Aufgabe, die der Erfindung zugrundeliegt.

Gegenstand der Erfindung ist also die Verwendung von 4-n-Butylresorcin zur Herstellung einer kosmetischen oder dermatologischen Zubereitung zur Prophylaxe oder Behandlung von Augenringen die durch eine gestörte Durchblutung der Haut im Augenbereich hervorgerufen werden.

4-n-Butylresorcin , CAS [18979-61-8], ist durch die chemische Struktur 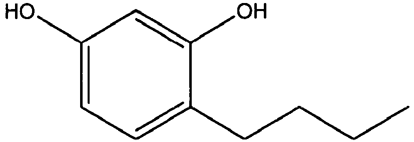 gekennzeichnet.

Bevorzugt enthalten kosmetische oder dermatologische Zubereitungen gemäß der Erfindung 0,001 - 10 Gew.-%, besonders bevorzugt 0,01 - 1 Gew.-%, an 4-n-Butylresorcin, bezogen auf die Gesamtzusammensetzung der Zubereitungen.

Zur Anwendung werden die kosmetischen und dermatologischen Zubereitungen erfindungsgemäß in der für Kosmetika üblichen Weise auf die Haut und/oder die Haare in ausreichender Menge aufgebracht.

Besonders bevorzugt sind solche kosmetischen und dermatologischen Zubereitungen, die in der Form eines Sonnenschutzmittels vorliegen. Vorteilhaft können diese zusätzlich mindestens einen weiteren UVA-Filter und/oder mindestens einen weiteren UVB-Filter und/oder mindestens ein anorganisches Pigment, bevorzugt ein anorganisches Mikropigment, enthalten.

Die kosmetischen und dermatologischen Zubereitungen können erfindungsgemäß kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an üblichen Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern die kosmetische oder dermatologische Zubereitung im Sinne der vorliegenden Erfindung eine Lösung oder Emulsion oder Dispersion darstellt, können als Lösungsmittel verwendet werden:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Sofern die kosmetische oder dermatologische Zubereitung im Sinne der vorliegenden Erfindung eine Lösung oder Emulsion oder Dispersion darstellt, können als Lösungsmittel verwendet werden:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Kosmetische Zubereitungen im Sinne der vorliegenden Erfindung können auch als Gele vorliegen, die neben einem wirksamen Gehalt am erfindungsgemäßen Wirkstoff und dafür üblicherweise verwendeten Lösungsmitteln, bevorzugt Wasser, noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise Bentonite, oder ein Gemisch aus Polyethylenglykol und Polyethylenglykolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Es ist erfindungsgemäß vorteilhaft, außer den erfindungsgemäßen Kombinationen weitere öllösliche UVA-Filter und/oder UVB-Filter in der Lipidphase und/oder weitere wasserlösliche UVA-Filter und/oder UVB-Filter in der wäßrigen Phase einzusetzen.

Vorteilhaft können die Lichtschutzformulierungen erfindungsgemäß weitere Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen. Sie können auch als Sonnenschutzmittel dienen.

Anhand des nachfolgenden Versuches soll die Wirksamkeit der vorliegenden Erfindung demonstriert werden.

Dieser Test zeigt die Aktivierung von Endothelzellen, wie sie z. B im entzündeten oder im gestressten Gewebe stattfindet. Dadurch weiten sich die Gefäße und der Blutfluss im Gewebe wird herabgesetzt, damit Entzündungszellen (in diesem Experiment Granulozyten) ins umgebene Gewebe auswandern können. Bei der dünnen Haut im Augenbereich äußert sich das durch sichtbare Augenringe. Eine verminderte Aktivierung des Gefäßendothels wirkt deshalb den Augenringen entgegen.

### Methodenbeschreibung:

Mikrovaskuläre Endothelzellen werden bis zur Konfluenz in 6-Well Schalen kultiviert. Die Zellen werden über Nacht mit dem Wirkstoff inkubiert und am nächsten morgen kommt TNFa als Stimulans dazu. Ein Well bleibt als Kontrolle ohne TNFa und ohne Wirkstoff. TNF inkubiert ca. 6 Stunden auf den Zellen. Wahrend dessen werden Granulozyten aus Buffy Coats (Blut) isoliert. Die Granulozyten werden auf die Endothelzellen gegeben und für 1 Stunde auf dem Schüttler inkubiert. Danach werden sie wieder abgewaschen und die Zellen fixiert.

Für die Auswertung wird die Zahl angehefteter Granulozyten am Mikroskop ausgewertet. In der Kontrolle sollte wenig anheften. Mit TNF relativ viel und wenn zusätzlich Wirkstoffe dazu kommen, dann sollten es auch weniger angeheftete Zellen sein.

Durch TNFa werden Adhäsionsmoleküle exprimiert, die die Anheftung der Granulozyten bewirken.

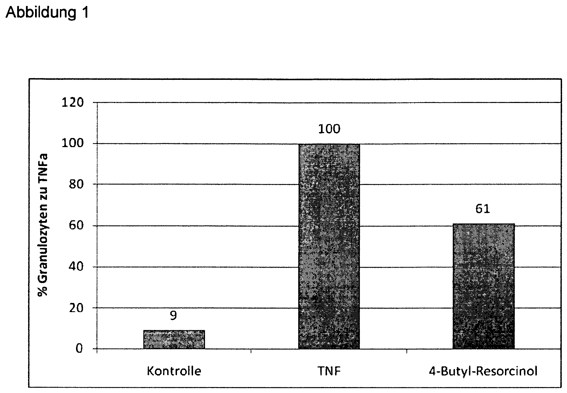

### Siehe Abbildung 1

| Rohstoff (INCI-Bezeichnung) | Gew.-% |
|---|---|
| Methylparaben | 0.2 |
| Propylparaben | 0.1 |
| Glyceryl Stearat | 3 |
| Caprylic/Capric Triglycerid | 4 |
| Cetearyl Alkohol | 3 |
| PEG-40 Stearat | 1 |
| Phenoxyethanol | 0.4 |
| Distärkephosphat | 1 |
| Natrium-Ascorbinsäurephosphat | 0,2 |
| C12-15 Alkyl Benzoat | 5 |
| Glycerin | 5 |
| Wasser + Natrium Hydroxid | 0.02 |
| Butylen Glykol | 3 |
| Natriummetabisulfit | 0.15 |
| Carbomer | 0.1 |
| Carraqeenan | 0.2 |
| Dicaprylyl Carbonat | 2.5 |
| 4-Butyl-Resorcin | 0.3 |
| Wasser | Ad 100.00 |
| PEG-40 Stearat | 1,0 |
| Glyceryl Stearat | 2,0 |
| Cera Microcristallina + Paraffinum Liqidum | 2 |
| Cetearyl Alkohol | 2 |
| Hydrogenated Vegetable Oil | 1 |
| Octyldodecanol | 2,0 |
| Caprylic/Capric Triglyceride | 2,0 |
| C12-15 Alkyl Benzoat | 2,0 |
| Tapioca Stärke+ Wasser | 1,0 |
| Trinatrium EDTA + Wasser | 1,0 |
| Methylparaben | 0,1 |
| Propylparaben | 0,1 |
| Phenoxyethanol | 0,3 |
| 4-Butyl-Resorcin | 0,3 |
| Süßholzextrakt (glycyrrhiza glabra) | 0,2 |
| Glycerin | 5,0 |
| Dimethicon | 1,0 |
| Cyclomethicon | 2,0 |
| Acrylates/C10-30 Alkyl Acrylate Crosspolymer | 0,2 |
| Xanthan Gum | 0,3 |
| Methylpropanediol | 2,0 |
| Tocopherylacetat | 0,5 |
| Natriumhydroxide | q.s. |
| Wasser | Ad 100 |

| Rohstoff (INCI-Bezeichnung) | Gew.-% |
|---|---|
| PEG-40 Stearat | 1,5 |
| Glyceryl Stearat | 3,0 |
| Dicaprylyl Carbonat | 5,0 |
| Caprylic/Capric Triglycerid | 4,0 |
| Coenzym Q10 (Ubichinon) | 0,2 |
| Cyclomethicon | 2,0 |
| C12-15 Alkyl Benzoat | 2,5 |
| Cetearyl Alcohol | 3,0 |
| Phenoxyethanol | 0,4 |
| Methylparaben | 0,2 |
| Propylparaben | 0,1 |
| Glycerin | 8,0 |
| 4-Butyl-Resorcin | 1,0 |
| Sodium Metabisulfit | 0,1 |
| Diethylhexyl Syringylidenmalonate + Caprylic/Capric Triglyceride | 0,1 |
| Natrium Polyacrylat | 0,2 |
| Carbomer | 0,1 |
| Natrium Hydroxide | q.s. |
| Wasser | ad 100 |

| Rohstoff (INCI-Bezeichnung) | Gew.-% |
|---|---|
| Polyglyceryl-3 Methylglucose Distearat | 2 |
| Sorbitan Stearat | 2 |
| TiO₂ (gecoated) | 2 |
| Hydrogenated Coco-Glycerides | 2 |
| Dicaprylyl Carbonat | 4 |
| Caprylic/Capric Triglycerid | 5 |
| Glycerin | 6 |
| Hexandiol | 2 |
| Vitamin A Palmitat | 0,05 |
| Methylparaben | 0,3 |
| Phenoxyethanol | 0,3 |
| 4-Butyl-Resorcin | 0,1 |
| Panthenol | 0,50 |
| Trinatrium EDTA + Wasser | 1 |
| Dioic acid | 0,1 |
| Acrylates/C10-30 Alkyl Acrylate Crosspolymer | 0,3 |
| Parfum | q.s. |
| Natrium Hydroxide | q.s. |
| Wasser | Ad 100 |

| Rohstoff (INCI-Bezeichnung) | Gew.% |
|---|---|
| | Gew.-% |
| Glyceryl Stearate Citrate | 2,0 |
| Behenyl Alcohol | 3,0 |
| Stearyl Alcohol | 2,0 |
| Cetearyl Alcohol | - |
| Butyl Methoxydibenzoylmethane | 2,0 |
| Phenylbenzimidazole Sulfonic Acid | 2,0 |
| Glycerin | 5,0 |
| Butylene Glycol | - |
| Dicaprylyl Ether | 4,0 |
| Caprylic/Capric Triglycerid | 6,0 |
| Octyldodecanol | - |
| Myristyl Myristat | - |
| C12-15 Alkyl Benzoat | - |
| Dicaprylyl Carbonat | - |
| Dimethicon | - |
| 4-Butyl-Resorcin | 0,5 |
| Distarch phosphate | 1,0 |
| Trisodium EDTA + Aqua | 1 |
| Phenoxyethanol | 0,3 |
| DMDM Hydantoin | 0,3 |
| Methylparaben | - |
| 2-Ethylhexyl-2-cyano-3-diphenylacrylat Ethylhexylmethoxycinnamat | 2,0 |
| Ammonium Acryloyldimethyltaurate/VP Copolymer | - |
| Carbomer | 0,4 |
| Natrium Hydroxide | q.s. |
| | q.s. |
| Wasser | Ad |
| 100 | Ad |
| 100 | |

## Patentansprüche

1. Dermatologische Zubereitungen mit einem Gehalt an 4-n-Butylresorcin zur Verwendung in der Prophylaxe oder Behandlung von Augenringen, die
a) nicht auf Folgen einer Pigmentstörung sondern
b) durch eine gestörte Durchblutung der Haut im Augenbereich hervorgerufen werden.

2. Zubereitungen zur Verwendung in der Prophylaxe oder Behandlung von Augenringen nach Anspruch 1, **dadurch gekennzeichnet, dass** sie 0,001 bis 10 Gew.-%, besonders bevorzugt 0,01 - 1 Gew.-%, an 4-n-Butylresorcin enthalten, bezogen auf die Gesamtzusammensetzung der Zubereitungen.

## Claims

1. Dermatological preparations having a content of 4-n-butylresorcinol for use in the prophylaxis or treatment of periorbital dark circles caused
a) not on a consequence of a pigment disorder, but
b) by an impaired blood circulation in the skin in the eye area.

2. Preparations for use in the prophylaxis or treatment of periorbital dark circles according to Claim 1, **characterized in that** they contain from 0.001 to 10% by weight, particularly preferably 0.01-1 % by weight, of 4-n-butylresorcinol, based on the total composition of the preparations.

## Revendications

1. Préparations dermatologiques ayant une teneur en 4-n-butylrésorcine, destinées à une utilisation dans la prophylaxie ou le traitement des cernes qui
a) ne sont pas sur la conséquence d'un trouble de la pigmentation, mais
b) qui sont plutôt causées par une perturbation de la circulation sanguine de la peau dans la zone de l'oeil.

2. Préparations destinées à une utilisation dans la prophylaxie ou le traitement des cernes selon la revendication 1, **caractérisées en ce qu'**elles contiennent 0,001 à 10 % en poids, de manière particulièrement préférée 0,01 à 1 % en poids, de 4-n-butylrésorcine, par rapport à la composition totale des préparations.
